# EUROPEAN PATENT APPLICATION

(11) **EP 0 986 996 A2**
(43) Date of publication of application: **22.03.2000**
(21) Application number: 99118393.0
(22) Date of filing: 16.09.1999
(51) Int. Cl.: A61F 13/15, A61F 15/00

(54) **Package for sanitary napkins**

(30) Priority: 17.09.1998 US 156780
(71) Applicant: McNeil-PPC, Inc., Skillman, NJ 08558-9418 (US)
(72) Inventor: Brown, Lisa Lynne, Princeton,NJ 08540 (US)
(74) Representative: Groening, Hans Wilhelm, Dipl.-Ing.

(57) **Abstract**

The present invention relates to a package for sanitary napkins that distinctly point out the size and shape of the actual product contained therein, so that a user can chose a product that accurately fits both their physical and psychological needs. The package comprises a means for determining the napkin's surface area, geometry, and thickness, without opening the package and exposing the napkins.

## Description

### Field of the Invention

The present invention relates to a package for sanitary napkins, whereby a user can determine the actual size and geometry of the napkins from the package, without having to open the package and exposing the napkins contained therein. It is most useful for determining the surface area and geometry of the napkin, whereby a user can determine the amount of undergarment coverage provided by the napkin.

### Background of the Invention

Convenient and discreet packaging of sanitary napkins has evolved over many decades. For convenience, many sanitary napkins are packaged in both primary and secondary units. The primary packaging unit may consist of an individually folded and wrapped napkin, enabling a user to discreetly transport napkins, preserving their hygiene and occupying less space, while the secondary packaging unit is that which protects the primary packaged napkins while they are shipped to retailers and reside on the shelf for sale. An example of an individually folded and wrapped napkin is disclosed in US Patent Number 4,556,146.

The secondary packaging typically contains informative and attractive graphics, while striving to maintain a high level of discreetness. A shortcoming of this approach, however, is that a user may not fully appreciate the napkin's geometry and size, until the package is opened and the napkins exposed. If the napkins once exposed do not meet a user's needs, then the napkins may be wasted.

Marketing of these products has revealed many variations of secondary packaging materials and forms. An example of one form is a flexible polymeric bag. The polymeric films making up the bags exist in a plethora of colors, including transparent films. However, the napkins contained in the transparent bags, are either folded or folded and wrapped, which hinder the ability to determine the true size and shape of the products.

In an effort to provide more information and guidance to users when they purchase feminine hygiene products, a method has been developed for the selection and use of a system of feminine hygiene products. The system, disclosed in PCT WO 97/45088, includes an information collection step, a system selection step, and an information provision step. Each system is comprised of at least two different feminine hygiene products.

The information collection step includes collecting information about a consumer's body size and menstrual cycle characteristics. WO 97/45088 discloses that the provision of information may be provided through the use of packaging indicia, such as a unique color, number, symbol, or some combination thereof.

A number of shortcomings still exist with the system disclosed in WO 97/45088. One of the shortcomings of the system is that a user is still required to guess what products are inside the package that will become available to her once purchased and removed from the package. A second shortcoming is that the consumer is placed into a category for the selection of products based on two variables, body type and menstrual cycle characteristics, which are subjectively determined and may result in the purchase of a product that is less than ideally suited for the consumer's actual needs.

There are commercially available sanitary napkins which are designed to manage a limited amount of bodily fluids, and the products can fail for a number of reasons besides saturation; therefore, a user considers undergarment coverage as important as, if not more important, than absorptive potential, for these types of napkins. In addition to providing a "comfort level" to a user with a certain amount of coverage, there is a user group that has larger body and undergarment dimensions compared to a user group having an average size body, that choose a product for its size, and not simply for its fluid absorbing capacity. For other consumers, who are smaller in size, a similar knowledge of the dimensions of the sanitary napkin might prove important to prevent them from purchasing a product that is too large for their body or undergarment dimensions and would therefor be unwieldy or uncomfortable.

One readily appreciates that there is still a need for a package of sanitary napkins to distinctly point out the size and shape of the actual product contained therein, so that a user can choose a product that accurately fits both their physical and psychological needs.

### Summary of the Invention

In accordance with the present invention, there has now been provided a package for sanitary napkins comprising a means for determining the napkin's surface area and thickness, without opening the package and exposing the napkins contained therein. One embodiment of the present invention is a package for sanitary napkins comprises a complete footprint or outline of the napkin's actual size on the outwardly disposed surface of the package, whereby the user can determine the amount of undergarment coverage provided by the napkin.

A second embodiment of the present invention is a package for sanitary napkins comprising a tab integral with the package, the tab having dimensions and geometry, which correspond to that of the sanitary napkins contained by the package.

A third embodiment of the present invention is a package for sanitary napkins comprising a sheet, which is releasably attached to one or more outwardly disposed surfaces of the package. The sheet has dimensions and geometry that correspond to that of the sanitary napkins contained by the package. A user can remove the sheet and hold it in her hands to better comprehend whether or not the napkins will meet her needs.

A fourth embodiment is a package for sanitary napkin comprising a weakened region with a perimeter and geometry that corresponds to that of the napkins contained by the package. The weakened region allows partial or whole separation of the portion of the package defined by the weakened region, from the remainder of the package, to provide access to the napkins contained therein.

In accordance with the present invention, there has also been provided a process of making a package for sanitary napkins, comprising the following steps: providing flexible packaging material; encasing at least one sanitary napkin with the flexible packaging material, the napkin having a size and geometry; evacuating air from within the encasing; and sealing the encasing, thereby, making a package that substantially corresponds to the size and geometry of the napkins contained therein.

Additionally, the present invention provides a dispenser for sanitary napkins comprising a means for determining the napkin's surface area, geometry, and thickness.

### Brief Description of the Figures

Figures 1a and 1b are perspective views of a rectangular sanitary napkin package with a footprint of the napkin's size and geometry on its outer surface panels.
Figure 2 is a perspective view of a sanitary napkin package with a tab, wherein the tab represents the napkin's dimensions and geometry.
Figures 3a and 3b are front views of a sanitary napkin package with a releasably adhered sheet hilly attached and partially attached to the outer surface of the package, respectively.

### Detailed Description of the Invention

The present invention relates to a package for sanitary napkins, whereby a user can determine the actual size and geometry of the napkins from the package, without the need to open the package and expose the napkins contained therein. Specifically, the present invention provides a package that enables a user to determine the surface area, thickness, and geometry of sanitary napkins from the exterior of the closed package. The surface area of a sanitary napkin directly relates to the amount of coverage the napkin provides to a user's undergarment and contact with a user's perineum. The thickness and geometry of a napkin has fluid management and comfort implications to a user.

Surface area as used in this specification and in the appended claims means the area of the body-facing surface of a sanitary napkin, and one surface of optional lateral extensions accompanying the sanitary napkin. In contrast, it does not mean the total surface area, which would include the body-facing surfaces, garment-facing surfaces, and outer side surfaces as determined by the distance between the body-facing and garment-facing surfaces of the napkin. As used in this specification and in the appended claims, footprint is understood to be the complete representation of the napkin's perimeter with true dimensions and geometry.

The description of the present invention will use the term sanitary napkin, which includes any article designed and used for collecting bodily fluids discharged from the cervix and urethra. These articles generally have a length greater than its width and have longitudinal edges, which are provided along the length of the article. The opposite edges, or the transverse edges, are shorter in dimension than the longitudinal edges. The sanitary napkin although elongated, may have varied forms, such as a rectangle, quadrilateral, quadrangle, an hourglass, or any of the numerous shapes and dimensions known in the art. The sanitary napkins are relatively thin articles, no thicker than about 5 centimeters, which have a body-facing surface, which is fluid permeable and a garment-facing surface, which is fluid impermeable.

Many of the incremental changes that have evolved in the development of new sanitary napkins are targeted to improve the protection offered by the products. One improvement is lateral extensions, commonly referred to as wings, which are well known in the art, see for example US Patent Number 4,900,320, hereby incorporated by reference.. Lateral extensions accompany many products in an effort to reduce leakage, and to keep the sanitary napkin in the location it was originally placed. Lateral extensions can be both flexible and stiff, can contain adhesive or not, can be wrapped around the underside of undergarments, can attach to the underside of undergarments, or can be held against or attached directly to the body.

The sanitary napkins can be individually folded and wrapped within the secondary packaging, an example of which is disclosed in US Patent Number 4,556,146. The present invention is particularly useful for these types of products because the secondary packaging can be constructed in a plurality of shapes and dimensions, which are not limited to the in-use form of the sanitary napkin, thereby lacking any indication of what the contained napkins actually provide.

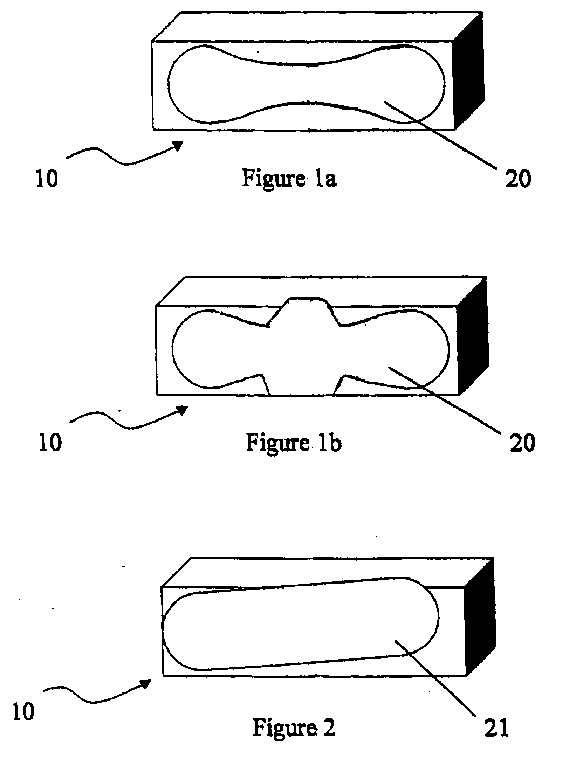

The following is a detailed description of the invention depicted in the Figures. Figure 1a shows a rectangular package 10 for sanitary napkins with a footprint 20, representing the sanitary napkins contained within the package, on the outer surface of one of the package's panels. The footprint 20, shown in Figure 1a, is of an hourglass shaped napkin; however, the utility of the present invention is not limited to this particular napkin or package geometry. Figure 1b shows a rectangular package 10 for sanitary napkins with a footprint 20, representing sanitary napkins with lateral extensions.

Methods to make the footprint on the outer surface of the package include, but is not limited to pigmentation, embossing, reverse embossing and adhering additional material. The footprint 20 is an integral part of the packaging material, and is not a separate, self supporting structure.

Figure 1a shows the footprint contained in a single plane of the package. Figure 1b shows the footprint preferably extending into multiple planes and multiple panels of the package in order for the complete outline to be accurately represented. Footprint extension into multiple packaging panels may also be required when the napkins are folded in manner, that the package containing the folded napkins does not have dimensions sufficient to represent the actual size of the napkin on a single planar surface.

Figure 2 depicts a package 10 for sanitary napkins with a tab 21 integral with the package. The tab 21 is a representation of the napkin, with the napkin's actual dimensions and geometry. The thickness of the tab may preferably represent the thickness of the napkin. The tab can be formed at the same time the package is formed, or can alternatively be formed separately and attached to the package. The tab can be constructed from similar or dissimilar materials to that of the package. Preferably, the tab is attached to the package at a single point, thereby allowing a user to partially disassociate the napkin representation from the package by rotating the tab away form the package, in order to get a better visualization and feel for the size and shape of the napkin. The tab can be attached to a package by means known in the art, such as heat seal, ultrasonics, sewn, adhesives and the like.

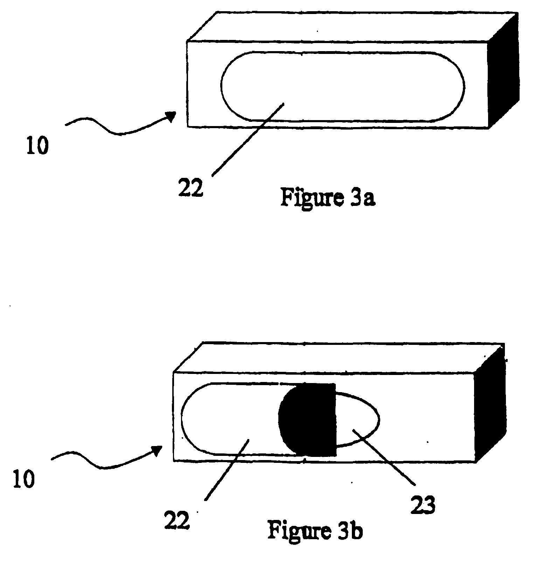

Figures 3a and 3b show front views of a sanitary napkin package 10 with a releasably adhered sheet 22 fully attached to the outer surface of the package and partially attached to the outer surface of the package respectively. The sheet 22 is a representation of the napkin contained within the package, with accurate surface area dimensions and geometry.

Optionally a region 23 of the package 10 that is covered by the sheet 22 is transparent, so that when the sheet is at least partially removed, the napkins contained within the package can be visualized. The visualization allows a user to see the actual product, the product's features and its materials, compared to that of an artist's rendition as typically shown on the outer surface of sanitary napkin packages; while maintaining the discreetness if desired by either not removing or refastening the sheet.

The sheet is preferably attached to the package with pressure-sensitive adhesive and can be refastened if removed. A representative, non-limiting list of adhesives useful for attaching the sheet to the package includes acrylics, starch based hot melts, adhesives based on block copolymers of vinyl aromatic hydrocarbon and one or more conjugated diene or hydrogenated aliphatic blocks, polylactic acids, and hot melts based on polyolefins, such as amorphous poly alpha olefings. The adhesive can be coated on either the sheet or the region of the package the sheet covers. To facilitate easy removal of the sheet from the package and minimize adhesive transfer, one of either the sheet or package can be coated with a release agent, such as silicone oil and the like.

Elements 20, 21 and 22, of Figures 1-3, illustrate different embodiments of the present invention in their method of connection to the package and the ability to further manipulate them, ranging from integral with the package to totally separable from the package. All of the different embodiments provide a means for indicating the surface area and geometry of sanitary napkins contained by the package, whereby a user can determine the amount of undergarment coverage provided by the napkins.

The present invention also relates to a process of making a package for sanitary napkins, wherein the resulting package will itself be an indication of the size and shape of the napkins contained therein. The process comprises removing all excess air from the non-sealed package, so that the packaging material will collapse onto the encased napkins, and therefore take the size and shape of the napkins. After the air is removed, the package is sealed, thereby preventing air from returning to the package. One technique for removing the air from the package is by the use of vacuum. Another technique is by applying normal force to the package. Vacuum and sealing is the preferred technique of the process, and may be performed at more than one location along the perimeter of the package, so as to minimize product and package distortion.

The disclosure thus far has focused on packages for sanitary napkins and processes of making the same. A similar benefit can be realized for a dispenser for sanitary napkins comprising a means for determining the napkin's surface area, thickness, and geometry on the exterior of the dispenser, or a part that is associated with the exterior of the dispenser. Dispensers of sanitary napkins are typically located in public restrooms and locker rooms. The means for determining the sanitary napkin surface area, the thickness and geometry on the exterior of the dispenser can include a footprint located on the outer surface of the dispenser; or a sample product adhered to the outer surface of the dispenser. Another effective way to communicate to the consumer the size and geometry of the sanitary napkin is to create a currency receiver, suitable for receiving either coins or paper bills, having the true dimensions and geometry of the contained napkins. In yet another embodiment, a disposal bag in the shape of the contained napkins, located on the exterior of the dispenser may be provided for disposal of any soiled napkins.

Packages for sanitary napkin products are typically constructed as either cartons or flexible packages, such as pouches and bags. Standard materials used to construct packages, include but are not limited to paperboard; polymeric film, such as polypropylene films, polyethylene films, co-extruded polyethylene and ethylene vinyl acetate films and the like; and coated paper. A package is formed through manipulation of a single sheet of material, such as folding, folding and sealing portions, by adhering multiple sheets to one another or a combination thereof The package is sealed or adhered by means known in the art, such as heat seal, ultrasonics, adhesives, hook and loop fasteners, and the like. Preferably, paperboard and adhesives are used to construct a carton package of the present invention.

Packaging systems for commodity products are well known in the art. US Patent Numbers 3,554,434; 4,246,286; 4,782,951; and 5,694,746, all of which are incorporated by reference herein, disclose a number of paperboard and flexible material packages and methods of making the same.

The package can optionally have opening and closure means to enable a user to easily retrieve individual sanitary napkins as needed and then close the package to keep the napkins clean and discreetly contained. The opening and closure means can include, but are not limited to flaps activated by applying force to lines of weakening; pursing systems, such as with string; pressure sensitive adhesives; hot melt adhesives; hook and loop fasteners; tab and slit; and interlocking rib and groove strips. Preferably a paperboard carton with a tab and slit closure means is employed in the present invention.

One of the embodiments of the present invention, not shown in the figures, is a package for sanitary napkins comprising a weakened region, wherein the region has a perimeter and geometry that corresponds to that of the napkins contained by the package. The lines of weakening can be made by scoring, perforating, embossing, or like techniques. A user can easily manipulate the weakened region to create a partial or complete opening in the package. If the region is only partially opened, then a flap is formed, which can then be re-associated with the remainder of the package, thereby discreetly protecting the napkins. The re-association can take place by using typical adhering means such as adhesives, hook and loop, bending, folding, and tucking, and other mechanical and chemical means.

The invention has been illustrated by, but is not intended to be limited to, the above description and figures. The scope of the invention is to be determined by the claims attached hereto.

## Claims

1. A package for sanitary napkins comprising sanitary napkins contained therein, and a means for indicating the surface area and geometry of the sanitary napkins integral with the outwardly disposed surface of the package, whereby a user can determine the amount of undergarment coverage provided by the sanitary napkins.

2. The package of claim 1 wherein the sanitary napkins are individually folded and wrapped.

3. The package of claim 1 further comprising a means for indicating the thickness of the sanitary napkin, integral with its outwardly disposed surface.

4. The package of claim 1 wherein the means for indicating the surface area and geometry of the sanitary napkins is a tab, having dimensions and geometry which correspond to that of the sanitary napkins.

5. The package of claim 4 wherein a region of the package underlying the tab is transparent.

6. The package of claim 1 wherein the means for indicating the surface area and geometry of the sanitary napkins is a releasably affixed sheet, having dimensions and geometry that correspond to that of the sanitary napkins.

7. The package of claim 6 wherein a region of the package in connection with the sheet is transparent.

8. The package of claim 1 wherein the means for indicating the surface area and geometry of the sanitary napkins is a weakened region, having a perimeter and geometry that corresponds to that of the sanitary napkins.

9. A process of making a sanitary napkin package, comprising the following steps:
a) providing flexible packaging material;
b) encasing at least one sanitary napkin with the flexible packaging material, the napkin having a size and geometry;
c) evacuating air from within the encasing; and
d) sealing the encasing,
thereby, making a package that substantially corresponds to the size and geometry of the napkins contained therein.

10. A dispenser for sanitary napkins comprising sanitary napkins contained therein, and a means integral with the dispenser for indicating the surface area and geometry of the sanitary napkins, whereby a user can determine the amount of undergarment coverage provided by the napkins.
